# FASCICULE DE BREVET EUROPEEN

(11) **EP 2 729 207 B1**
(45) Date de publication et mention de la délivrance du brevet: **26.08.2015**
(21) Numéro de dépôt: 11773488.9
(22) Date de dépôt: 07.09.2011
(51) Int. Cl.: A61M 15/00

(54) **INHALATEUR DE POUDRE SECHE**
TROCKENPULVERINHALATOR
DRY POWDER INHALER

(30) Priorité: 05.07.2011 FR 1156039
(43) Date de publication de la demande: 14.05.2014
(73) Titulaire: Aptar France SAS, 27110 Le Neubourg (FR)
(72) Inventeur: BAILLET, Matthieu, 76240 Bonsecours (FR); LAUT, Antoine, 95420 Wy dit Joli Village (FR)
(74) Mandataire: CAPRI
(86) Numéro de dépôt international: PCT/FR2011/052040
(87) Numéro de publication internationale: WO 2013/004921

(56) Documents cités:
- EP-A2- 0 271 029
- DE-A1- 19 742 994
- FR-A1- 2 228 499
- GB-A- 2 061 735
- US-A- 4 206 758
- US-A- 4 353 365
- US-A1- 2004 255 940

## Description

La présente invention concerne un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Concernant l'ouverture des réservoirs individuels, il a été proposé de peler ou décoller la couche de fermeture. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Un autre problème qui se pose avec les inhalateurs pourvus de bande de blisters est lié au déplacement de la bande, et au stockage de la partie utilisée de la bande. Ainsi, selon la longueur de la bande et/ou l'épaisseur des blisters, un espace important peut s'avérer nécessaire et tout blocage de la bande de blisters peut empêcher le bon fonctionnement de l'inhalateur. Par ailleurs, lorsque le dispositif d'avancée de la bande tire en même temps sur l'extrémité avant de la bande pour éviter un mauvais enroulement, il peut se poser un problème au fur et à mesure des actionnements en raison notamment du diamètre de la bande usée enroulée qui augmente progressivement. Les inhalateurs multidoses et les inhalateurs contenant une bande de blisters sont donc généralement des dispositifs complexes, constituées d'un grand nombre de pièces, et donc coûteux à fabriquer et à assembler. Pour réaliser des dispositifs moins complexes et donc moins coûteux, il a été proposé des inhalateurs comportant des réservoirs individuels, tels que des capsules ou gélules, qui sont à charger dans l'inhalateur juste avant l'utilisation de celui-ci. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. De plus, d'autres inconvénients spécifiques à ces inhalateurs à capsules sont apparus. Ainsi, ces dispositifs sont généralement constitués de deux pièces, l'une étant pourvue de l'embout buccal. Lors de la manipulation de ces dispositifs, pour ouvrir la capsule et libérer la poudre, ou pour vider la capsule vide après inhalation, les doigts de l'utilisateur entrent généralement en contact avec l'embout buccal, ce qui peut présenter des risques de contamination. De plus, dans ce type de dispositifs, la partie supérieure de la capsule reste généralement coincée dans l'ouverture de chargement après ouverture de la capsule, généralement réalisée par séparation de la partie de capsule inférieure. Ce n'est que lorsque l'utilisateur introduit la prochaine capsule dans l'ouverture de chargement qu'il va expulser cette partie supérieure. Ceci peut présenter des inconvénients, notamment lorsqu'il se passe du temps entre deux utilisations du dispositif. Les documents US 2004/255940 A1, US 4 206 758 A, EP 0 271 029 A2, US 4 353 365 A et DE 197 42 994 A1 divulguent des inhalateurs de poudre sèche selon l'art antérieur. Le document US 2004/255940 A1 divulgue un inhalateur selon le préambule de la revendication 1.

La présente invention a pour but de fournir un inhalateur de poudre sèche, qui ne reproduit pas les inconvénients susmentionnés.

En particulier, la présente invention a pour but de fournir un tel inhalateur qui soit simple et peu coûteux à fabriquer et à assembler, fiable d'utilisation, et limitant autant que possible les risques de contamination et/ou de pollution.

La présente invention a donc pour objet un inhalateur de poudre sèche selon la revendication 1.

Avantageusement, ledit capuchon comporte une ouverture alignée avec l'ouverture de chargement de la molette en position de chargement de capsule.

Avantageusement, ladite ouverture est disposée au fond d'une cuvette facilitant l'insertion manuelle de la capsule dans l'ouverture de chargement de la molette.

Avantageusement, ladite molette est rotative dans les deux sens par rapport audit corps pour ouvrir ladite capsule.

Avantageusement, ladite chambre de dispersion comporte des entrées d'air permettant de créer des flux d'air supplémentaires lors de l'inhalation pour davantage faire tourbillonner la poudre dans la chambre de dispersion avant son inhalation.

Avantageusement, lesdites entrées d'air sont réalisées de manière environ tangentielle dans la paroi cylindrique du corps.

Avantageusement, ledit embout buccal est pourvu d'une grille pour empêcher l'expulsion des parties de capsules à travers ledit orifice de distribution.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique éclatée en perspective d'un dispositif de distribution selon un mode de réalisation avantageux de l'invention,
- les figures 2a et 2b sont des vues en perspective de dessus et de dessous du capuchon,
- les figures 3a et 3b sont des vues en perspective de dessus et de coté de la molette,
- les figures 4a et 4b sont des vues en perspective de coté et de dessus du corps,
- les figures 5a à 5d sont des vues schématiques en perspective de coté, en perspective découpée et en coupe longitudinale selon deux plans de coupe différents du dispositif, avant chargement de la capsule,
- la figure 5e est une vue en section transversale du dispositif de la figure 5a,
- la figure 5f est une vue de détail de la figure 5c,
- les figures 6a et 6b sont des vues similaires à celles des figures 5a et 5b, après chargement de la capsule,
- la figure 6c est une figure similaire à la figure 5d, après chargement de la capsule,
- la figure 6d est une vue partielle en perspective découpée similaire à celle de la figure 6b mais selon un autre angle de vue,
- les figures 7a et 7b sont des vues similaires à celles des figures 6b et 6d, en cours d'ouverture de la capsule,
- les figures 8a et 8b sont des vues similaires à celles des figures 7a et 7b, après ouverture de la partie inférieure de la capsule mais avant expulsion de la partie supérieure de la capsule,
- la figure 8c est une vue en section transversale dans la position des figures 8a et 8b,
- les figures 9a et 9b sont des vues similaires à celles des figures 6b et 6c, après expulsion de la partie supérieure de la capsule dans la chambre de dispersion,
- la figure 10 est une vue similaire à celle de la figure 5e, en cours d'inhalation, et
- les figures 11 a et 11 b sont des vues similaires à celles des figures 5a et 5b, montrant l'évacuation des parties de capsule vides.

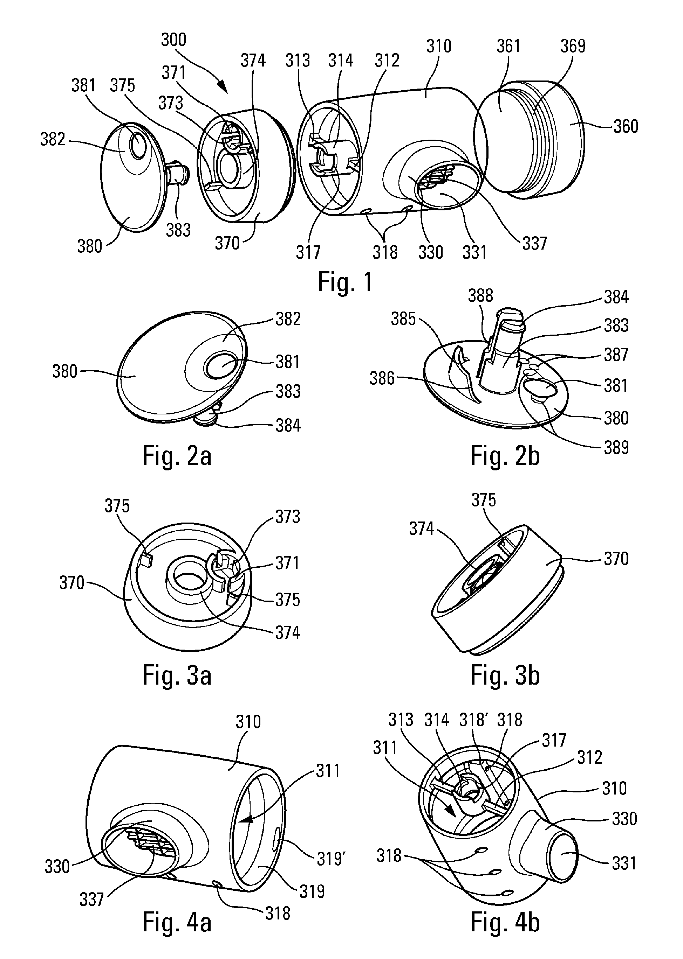

Les figures illustrent un mode de réalisation avantageux de la présente invention. La figure 1 illustre notamment une vue en perspective éclatée du dispositif. Dans ce mode de réalisation, l'inhalateur 300 n'est constitué que de quatre pièces. Le dispositif de l'invention est destiné à recevoir un capsule ou gélule 10, formées de deux parties 11, 12 emboitées l'une dans l'autre, et contenant une dose de poudre.

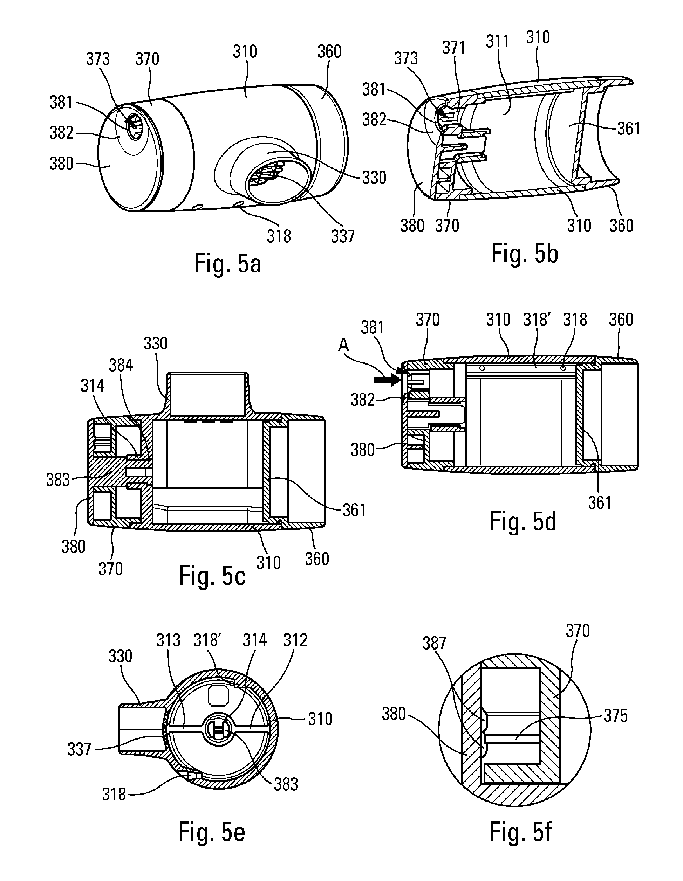

Un corps creux 310 de forme sensiblement cylindrique, par rapport à un axe longitudinal, est pourvu sur sa périphérie d'un embout buccal 330 définissant l'orifice de distribution 331. L'intérieur du corps 310 définit une chambre de dispersion 311. De préférence, le corps 310 est réalisé en matériau transparent ou translucide, avantageusement teinté, pour permettre de visualiser l'intérieur de la chambre de dispersion 311 depuis l'extérieur. Ce corps 310 est ouvert sur ces deux cotés axiaux. Sur le premier coté axial, le corps 310 comporte un manchon creux 314 central fixé au corps par deux entretoises latérales 312 et 313. Ces entretoises forment chacune un profil d'ouverture de capsule, comme cela sera explicité ci-après. Le manchon creux 314 comporte deux découpes axiales 317. Plusieurs trous de passage d'air 318 sont réalisés dans la paroi cylindrique du corps 310 pour créer des entrées d'air dans la chambre de dispersion 311. Ces entrées d'air sont environ tangentielles, et ont notamment pour but de favoriser la bonne distribution de la poudre à chaque inhalation. Avantageusement, cinq trous peuvent être prévus, dont trois sont visibles sur la figure 4b. Avantageusement, ces trous de passages 318 sont formés au niveau d'épaulements longitudinaux diamétralement opposés, formés dans la surface interne cylindrique du corps 310. Ces épaulements 318', visibles notamment sur les figures 5d et 5e, renforcent le corps au niveau des trous de passage et favorise le tourbillonnement dans la chambre de dispersion. Bien entendu, des entrées d'air différentes et un nombre quelconque d'entrées d'air sont aussi possibles.

Une molette 370 est fixée au corps 310 sur le premier coté axial de celui-ci, et un bouchon 360 est fixé sur le second coté axial. Cette molette 370 et ce bouchon 360 forment des première et seconde parties de préhension que l'utilisateur va manipuler pour utiliser le dispositif.

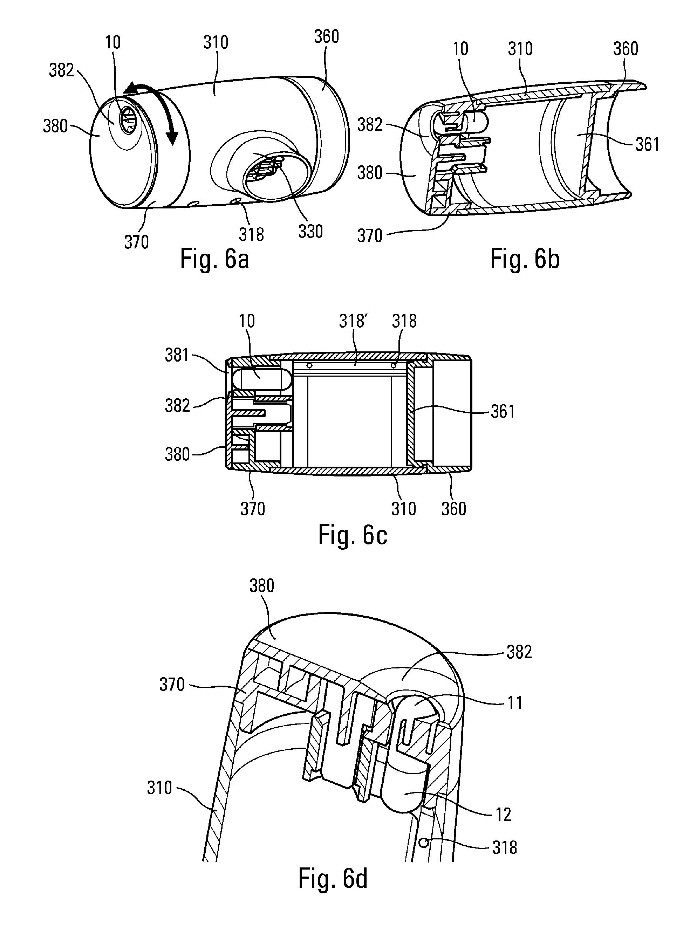

Les figures 3a et 3b illustrent plus particulièrement ladite molette 370. Avantageusement, cette molette 370 pourrait présenter un profil externe particulier, tel que des nervures ou rainures longitudinales, pour inciter l'utilisateur à manipuler le dispositif par son intermédiaire. Cette molette 370 comporte une ouverture de chargement 371 de capsule ou gélule 10. Cette ouverture de chargement 371 s'étend avantageusement axialement dans le sens de l'axe longitudinal du corps 310, et débouche dans ladite chambre de dispersion 311. La capsule est réalisée en deux parties, avec une partie supérieure 11 de capsule et une partie inférieure 12 de capsule. La capsule 10 est insérée dans l'ouverture de chargement 371 d'abord par sa partie inférieure 12, de sorte que lorsque la capsule 10 est en position chargée, comme représentée notamment sur la figure 6d, la partie inférieure 12 de capsule fait saillie dans la chambre de dispersion 311, alors que la partie supérieure 11 de capsule est coincée dans l'ouverture de chargement 371. Cette ouverture de chargement 371 est avantageusement pourvue de moyens de coincement, tels que des nervures 373, pour retenir la capsule 10, plus particulièrement la partie supérieure 11 de ladite capsule 10.

La molette 370 comporte un capuchon de fermeture 380 formant l'extrémité axiale du dispositif sur le premier coté axial. Ce capuchon de fermeture, mieux visible sur les figures 2a et 2b, comporte une ouverture 381 alignée en position de chargement de capsule sur l'ouverture de chargement 371. Avantageusement, cette ouverture 381 se situe dans une petite dépression 382, environ de la taille d'un doigt, qui facilite l'insertion complète de la capsule 10 par l'utilisateur. Sur sa surface interne, visible sur la figure 2b, le capuchon 380 comporte un plot central 383 saillant axialement, et qui se termine par un profil d'encliquetage approprié 384. Le plot central 383 comporte deux profils axiaux 388 destinés à coopérer avec les découpes axiales 317 du manchon creux 314 du corps 310. Ainsi, une fois assemblé, le capuchon 380 s'encliquète sur le corps, avec le profil d'encliquetage 384 du capuchon qui vient s'encliqueter dans le manchon creux 314 du corps. La coopération des profils axiaux 388 du capuchon avec les découpes axiales 317 du manchon creux solidarise en rotation le corps 310 et le capuchon 380. Ainsi, lorsque l'utilisateur fait tourner la molette 370 par rapport au corps 310, le capuchon 380 reste fixe en rotation par rapport audit corps, et la molette 370 tourne donc également par rapport audit capuchon 380. Le capuchon 380 comporte en outre un profil saillant 385 sur sa surface interne, ledit profil saillant 385 formant un arc de cercle. A chaque extrémité, ledit profil saillant 385 comporte une zone inclinée 386, comme visible sur la figure 2b. La molette comporte également avantageusement deux nervures d'indexation 375 adaptés à coopérer avec un profil d'indexation correspondant 387 formé sur la face interne du capuchon 380 pour assurer un bon positionnement du capuchon 380 sur la molette 370 en position de repos, avec un bord d'une nervure 375 de la molette coincé dans le profil 387 du capuchon 380, comme visible sur la figure 5f. Par ailleurs, la face interne du capuchon 380 peut aussi avantageusement comporter des moyens de positionnement 389 de la capsule, disposés au bord de l'ouverture 381. Ces moyens de positionnement 389 peuvent servir à repositionner la capsule 10 sur les premiers degrés de rotation de la molette 370, lorsque la capsule n'a pas été parfaitement positionnée lors de son insertion par l'utilisateur. On garantit ainsi qu'à chaque actionnement, la capsule 10 est parfaitement positionnée dans la molette 370 pour ainsi permettre une ouverture fiable de ladite capsule à chaque actionnement.

Le bouchon 360 forme la seconde partie d'extrémité axiale du corps. Ce bouchon 360 forme la paroi de fond 361 de la chambre de dispersion 311, et est amovible du corps 310 pour permettre l'évacuation des parties de capsule vides après chaque utilisation. Avantageusement, au moins la paroi de fond 361 est réalisée en un matériau transparent ou translucide, qui peut être teinté, de préférence le même matériau que le corps 310. De préférence, ce bouchon 360 est fixe en rotation par rapport au corps 310. Pour ce faire, le corps 310 peut comporter un profil interne 319 qui coopère par frottements avec un profil interne 369 du bouchon. Un ergot d'assemblage 319' peut aussi être prévu sur le corps 310 pour assurer une bonne fixation du bouchon. Eventuellement, le bouchon 360 peut présente un profil externe similaire à celui de la molette 370. L'utilisateur est donc naturellement incité à saisir chaque partie de préhension 360, 370 avec une main respective, et de faire tourner l'une par rapport à l'autre pour manipuler le dispositif. Ainsi, pour utiliser le dispositif de ce mode de réalisation, l'utilisateur agrippe avec ses deux mains les deux parties de préhension 360 et 370 et fait tourner l'une par rapport à l'autre, comme cela sera expliqué plus en détail ci-après. A aucun moment, l'utilisateur n'est obligé de toucher la partie d'embout buccal 330 pour utiliser le dispositif. En variante, le bouchon 360 pourrait être rotatif sur le corps. Dans ce cas, l'utilisateur devrait de préférence aussi saisir le bord axial du corps 310 lors de l'utilisation du dispositif, auquel cas le bouchon 360 pourrait être de largeur réduite.

Les figures 5 et 6 illustrent une phase de chargement et d'ouverture de capsule 10. Ainsi, en se référant plus particulièrement à la figure 5b, on voit une section à travers le corps 310 et donc on voit la chambre de dispersion 311, l'ouverture de chargement 371 et l'embout buccal 330. L'utilisateur charge une capsule 10 selon la flèche A sur la figure 5d dans l'ouverture de chargement 371. La profondeur dudit orifice 371 est ajustée pour que lorsque l'utilisateur insère entièrement la capsule 10 dans ladite ouverture de chargement 371, la partie supérieure 11 de la capsule est maintenue serrée dans ladite ouverture de chargement 371 alors que la partie inférieure 12 de la capsule se projette à l'intérieur de la chambre de dispersion 311.

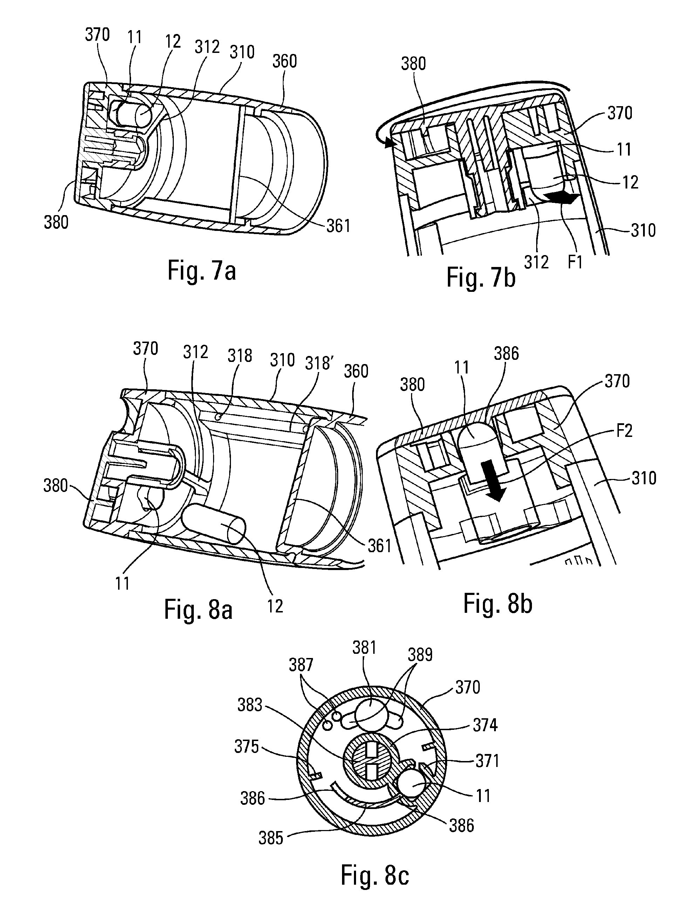

L'utilisateur va alors tourner la molette 370 par rapport au corps 310. Cette rotation est illustrée sur les figures 7 et 8. Comme représenté sur la figure 6a, cette rotation de la molette 370 peut se faire dans les deux sens par rapport au corps 310, de sorte que le dispositif est aussi bien utilisable par les droitiers que par les gauchers. Comme visible notamment sur la figure 7a, une des entretoises 312, 313 va venir en contact de la partie inférieure 12 de la capsule 10. La figure 7a montre la position juste avant que la capsule 10 ne soit ouverte alors que la figure 8a montre la capsule après ouverture, avec l'entretoise 312 qui a poussé sur la partie inférieure 12 de la capsule. On voit que la rotation de l'entretoise 312 a provoqué la déformation de la partie inférieure 12 de capsule, illustrée par la flèche F1 sur la figure 7b, laquelle va donc se séparer de la partie supérieure 11 de la capsule, qui reste coincée dans l'ouverture de chargement 371. La figure 8a illustre la position de la capsule 10 ouverte avec la partie supérieure 11 coincée dans l'ouverture de chargement 371 et la partie inférieure 12 qui est tombée librement dans la chambre de dispersion 311 pour se vider dans celle-ci. Une poursuite de la rotation de la molette 370 va alors amener le profil saillant 385 du capuchon en contact avec la partie supérieure 11 de capsule. Comme visible sur les figures 8b et 8c, la zone inclinée 386 de ce profil saillant va alors progressivement pousser la partie supérieure 11 de capsule, jusqu'à ce qu'elle tombe la chambre de dispersion 311.

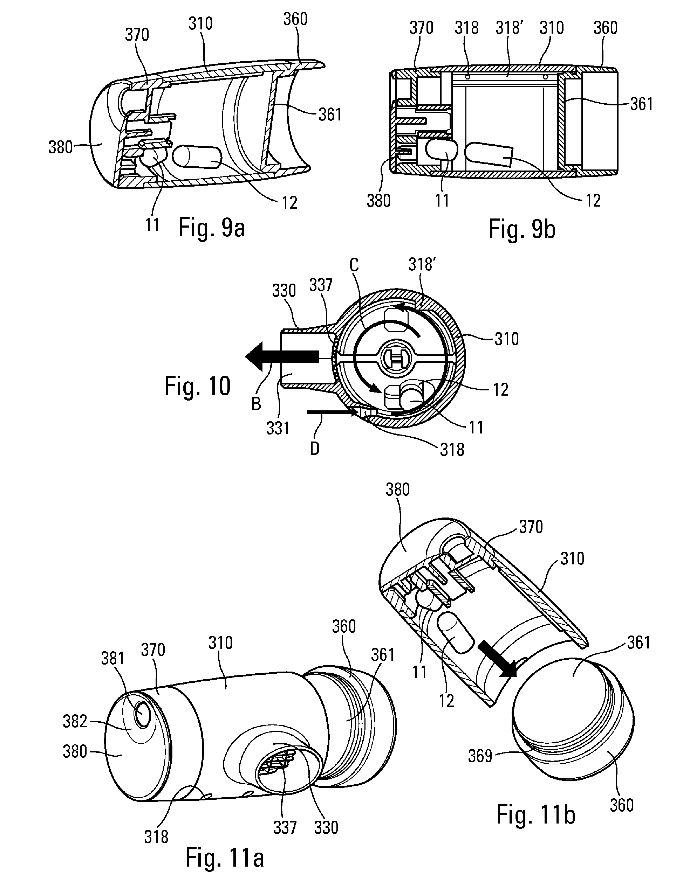

Les figures 9a et 9b illustrent le dispositif prêt pour l'inhalation, avec les deux parties de capsules 11 et 12 séparées et tombées dans la chambre de dispersion 311. L'utilisateur peut alors inhaler comme illustré par la flèche B sur la figure 10. Pour ce faire, il place sa bouche autour de l'embout buccal 330 et va créer un flux d'inhalation qui va lui permettre d'inhaler la poudre contenue dans la chambre de dispersion 311 à travers l'orifice de distribution 331. La forme sensiblement cylindrique de la chambre de dispersion 311 est avantageuse en ce qu'elle favorise le tourbillonnement du flux d'inhalation provenant de l'embout buccal. L'embout buccal comporte de préférence une grille 337 pour laisser passer la poudre mais empêcher les parties de capsule d'être expulsées dans la bouche de l'utilisateur. Le flux d'inhalation créé par l'utilisateur va faire tourbillonner les partie supérieure 11 et inférieure 12 de capsule, qui tournent librement à l'intérieur de la chambre de dispersion 311. Ceci assure le vidage des deux parties de capsule et permet une bonne dispersion et désagglomération de la poudre au moment de sa distribution à l'utilisateur. Ce tourbillonnement est illustré par la flèche C sur la figure 10. Les trous de passage 318 ménagés dans le corps 310 favorisent le tourbillonnement du flux d'inhalation puisque l'utilisateur, au moment où il inhale, va aspirer des flux d'air à travers ces trous de passage 318, comme illustré par la flèche D sur la figure 10, qui vont donc naturellement tourner à l'intérieur de ladite chambre de dispersion 311, et ainsi faire tourbillonner encore davantage les parties de capsule 11, 12, pour disperser et désagglomérer la poudre.

Après l'inhalation, l'utilisateur retire le bouchon 360 comme illustré sur les figures 11 a et 11 b, pour évacuer les parties de capsules 11, 12 de la chambre de dispersion 311. Il ramène aussi la molette en position de départ, avec l'ouverture 381 du capuchon 380 alignée avec l'ouverture de chargement 371 de la molette 370. Le dispositif est alors prêt pour la prochaine utilisation.

Le mode de réalisation de la présente invention décrit ci-dessus permet donc de réaliser un dispositif où l'utilisateur n'a pas besoin de manipuler l'embout buccal 330 pour utiliser le dispositif. Ce mode de réalisation est particulièrement simple, puisqu'il ne comporte que quatre pièces. Il permet de garantir une bonne dispersion de la poudre, d'une part en la désagglomérant de manière appropriée grâce aux parties de capsule qui tourbillonnent dans la chambre de dispersion, mais également grâce aux arrivées d'air supplémentaires ménagées dans le corps 310. Le procédé d'utilisation du dispositif est également très simple, l'utilisateur n'ayant qu'à déplacer la molette pour complètement actionner le dispositif. Ainsi, il insère d'abord une capsule, puis il fait tourner la molette, puis il inhale, et enfin il ramène la molette vers sa position de départ et vide les parties de capsule en retirant le bouchon.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Inhalateur de poudre sèche comportant un corps (310) sensiblement cylindrique définissant une chambre de dispersion (311), ledit corps (310) comportant sur sa paroi cylindrique un embout buccal (330) définissant un orifice de distribution (331), ledit corps (310) étant pourvu à sa première partie d'extrémité axiale d'une molette (370) montée rotative sur ledit corps (310), ladite molette (370) comportant une ouverture de chargement (371) pour recevoir une capsule (10) contenant un dose de poudre sèche, ledit corps (310) étant pourvu à sa seconde partie d'extrémité axiale d'un bouchon (360) montée de manière amovible sur ledit corps (310), ledit corps (310) comportant au moins un profil d'ouverture (312, 313) adapté à ouvrir une capsule (10) insérée dans ladite ouverture de chargement (371) lorsque ladite molette (370) est déplacée en rotation par rapport audit corps (310), **caractérisé en ce que** ladite molette (370) comporte un capuchon (380) monté fixement sur ledit corps (310) de telle sorte que ladite molette (370) est aussi rotative par rapport audit capuchon (380), ledit corps (310) comportant un manchon creux central (314) coopérant avec un profil d'encliquetage (384) d'un plot central (383) du capuchon (380) pour fixer ledit capuchon (380) audit corps (310), ledit manchon creux central (314) étant fixé à la paroi cylindrique du corps (310) par deux entretoises (312, 313) formant chacune un profil d'ouverture de capsule, ladite capsule (10) comportant une partie supérieure (11) de capsule et une partie inférieure (12) de capsule, lesdites deux parties de capsules étant disposées dans la chambre de dispersion (311) lors de l'inhalation, ledit capuchon (380) comportant un profil saillant (385) adapté à expulser la partie supérieure (11) de capsule de l'ouverture de chargement (371) de la molette (370) dans la chambre de dispersion (311) après ouverture de ladite capsule (10) par ledit profil d'ouverture (312, 313) du corps (310).

2. Inhalateur selon la revendication 1, dans lequel ledit capuchon (380) comporte une ouverture (381) alignée avec l'ouverture de chargement (371) de la molette (370) en position de chargement de capsule.

3. Inhalateur selon la revendication 2, dans lequel ladite ouverture (381) est disposée au fond d'une cuvette (382) facilitant l'insertion manuelle de la capsule dans l'ouverture de chargement (371) de la molette (370).

4. Inhalateur selon l'une quelconque des revendications précédentes, dans lequel ladite molette (370) est rotative dans les deux sens par rapport audit corps (310) pour ouvrir ladite capsule (10).

5. Inhalateur selon l'une quelconque des revendications précédentes, dans lequel ladite chambre de dispersion (311) comporte des entrées d'air (318) permettant de créer des flux d'air supplémentaires lors de l'inhalation pour davantage faire tourbillonner la poudre dans la chambre de dispersion avant son inhalation.

6. Inhalateur selon la revendication 5, dans lequel lesdites entrées d'air (318) sont réalisées de manière environ tangentielle dans la paroi cylindrique du corps (310).

7. Inhalateur selon l'une quelconque des revendications précédentes, dans lequel ledit embout buccal (330) est pourvu d'une grille (337) pour empêcher l'expulsion des parties de capsules à travers ledit orifice de distribution (331).

## Patentansprüche

1. Inhalator für ein Trockenpulver, umfassend einen im Wesentlichen zylindrischen Körper (310), der eine Verteilungskammer (311) definiert, wobei der Körper (310) an seiner zylindrischen Wand einen Mundansatz (330) umfasst, der eine Ausgabeöffnung (331) definiert, wobei der Körper (310) an seinem ersten axialen Endteil mit einem Rädchen (370) versehen ist, das drehbar auf dem Körper (310) montiert ist, wobei das Rädchen (370) eine Beschickungsöffnung (371) umfasst, um eine Kapsel (10) aufzunehmen, die eine Dosis von Trockenpulver enthält, wobei der Körper (310) an seinem zweiten axialen Endteil mit einem Abschluss (360) versehen ist, der lösbar an dem Körper (310) montiert ist, wobei der Körper (310) mindestens ein Öffnungsprofil (312, 313) umfasst, das dafür geeignet ist, eine Kapsel (10) zu öffnen, die in der Beschickungsöffnung (371) eingefügt ist, wenn das Rädchen (370) drehend in Bezug auf den Körper (310) verlagert wird, **dadurch gekennzeichnet, dass** das Rädchen (370) eine Kappe (380) umfasst, die derart fest auf dem Körper (310) montiert ist, dass das Rädchen (370) auch in Bezug auf die Kappe (380) drehbar ist, wobei der Körper (310) eine mittige hohle Hülse (314) umfasst, die mit einem Sperrprofil (384) einer mittigen Steckstelle (383) der Kappe (380) zusammenwirkt, um die Kappe (380) am Körper (310) zu befestigen, wobei die mittige hohle Hülse (314) an der zylindrischen Wand des Körpers (310) durch zwei Zwischenstücke (312, 313) befestigt ist, die jeweils ein Öffnungsprofil der Kapsel bilden, wobei die Kapsel (10) einen oberen Kapselteil (11) und einen unteren Kapselteil (12) umfasst, wobei die beiden Kapselteile bei der Inhalation in der Verteilungskammer (311) angeordnet sind, wobei die Kappe (380) ein vorspringendes Profil (385) umfasst, das dazu geeignet ist, den oberen Kapselteil (11) der Beschickungsöffnung (371) des Rädchens (370) in der Verteilungskammer (311) nach Öffnung der Kapsel (10) durch das Öffnungsprofil (312, 313) des Körpers (310) auszuwerfen.

2. Inhalator nach Anspruch 1, wobei die Kappe (380) eine Öffnung (381) umfasst, die in der Beschickungsposition der Kapsel mit der Beschickungsöffnung (371) des

3. Inhalator nach Anspruch 2, wobei die Öffnung (381) am Boden einer Mulde (382) angeordnet ist, die das manuelle Einführen der Kapsel in die Beschickungsöffnung (371) des Rädchens (370) erleichtert.

4. Inhalator nach einem der vorhergehenden Ansprüche, wobei das Rädchen (370) in beide Richtungen in Bezug auf den Körper (310) drehbar ist, um die Kapsel (10) zu öffnen.

5. Inhalator nach einem der vorhergehenden Ansprüche, wobei die Verteilungskammer (311) Lufteinlässe (318) umfasst, die es erlauben, bei der Inhalation zusätzliche Luftströme zu erzeugen, um das Pulver in der Verteilungskammer vor dessen Inhalation stärker aufzuwirbeln.

6. Inhalator nach Anspruch 5, wobei die Lufteinlässe (318) in etwa tangential in der zylindrischen Wand des Körpers (310) realisiert sind.

7. Inhalator nach einem der vorhergehenden Ansprüche, wobei der Mundansatz (330) mit einem Gitter (337) versehen ist, um das Ausstoßen von Kapselteilen durch die Ausgabeöffnung (331) zu verhindern.

## Claims

1. A dry powder inhaler comprising a body (310) that is substantially cylindrical and that defines a dispersion chamber (311), said body (310) including, on its cylindrical wall, a mouthpiece (310) that defines a dispenser orifice (331), said body (310) being provided, at its first axial end portion, with a knurled wheel (370) that is mounted to turn on said body (310), said knurled wheel (370) including a loading opening (371) for receiving a capsule (10) containing a dose of dry powder, said body (310) being provided, at its second axial end portion, with a stopper (360) that is mounted in removable manner on said body (310), said body (310) including at least one opening profile (312, 313) that is adapted to open a capsule (10) inserted in said loading opening (371) when said knurled wheel (370) is turned relative to said body (310), the inhaler being **characterized in that** said knurled wheel (370) includes a cap (380) that is mounted in stationary manner on said body (310) such that said knurled wheel (370) also turns relative to said cap (380), said body (310) including a central hollow sleeve (314) that co-operates with a snap-fastener profile (384) of a central pin (383) of the cap (380) for fastening said cap (380) to said body (310), said central hollow sleeve (314) being fastened to the cylindrical wall of the body (310) via two spacers (312, 313) that form a capsule-opening profile, said capsule (10) including a top capsule portion (11) and a bottom capsule portion (12), said two capsule portions being located in the dispersion chamber (311) during inhalation, said cap (380) including a projecting profile (385) that is adapted to expel the top capsule portion (11) from the loading opening (371) of the knurled wheel (370) into the dispersion chamber (311) after said capsule (10) has been opened by said opening profile (312, 313) of the body (310).

2. An inhaler according to claim 1, wherein said cap (380) includes an opening (381) that is in alignment with the loading opening (371) of the knurled wheel (370) in the capsule-loading position.

3. An inhaler according to claim 2, wherein said opening (381) is arranged at the bottom of an indentation (382) that makes it easier to insert the capsule manually into the loading opening (371) of the knurled wheel (370).

4. An inhaler according to any preceding claim, wherein said knurled wheel (370) may be turned in both directions relative to said body (310) in order to open said capsule (10).

5. An inhaler according to any preceding claim, wherein said dispersion chamber (311) includes air inlets (318) that make it possible to create additional flows of air during inhalation so as to enhance the swirling of the powder in the dispersion chamber before being inhaled.

6. An inhaler according to claim 5, wherein said air inlets (318) are formed in approximately tangential manner in the cylindrical wall of the body (310).

7. An inhaler according to any preceding claim, wherein said mouthpiece (330) is provided with a grid (337) so as to prevent the capsule portions from being expelled through said dispenser orifice (331).
